Europäisches Patentamt

European Patent Office

Office européen des brevets

(19)

(11) Publication number: **0 217 407 B1**

# EUROPEAN PATENT SPECIFICATION

(12)

(45) Date of publication of patent specification: **06.03.91**   (51) Int. Cl.5: **C07C 67/37**, C07C 69/587, B01J 27/08, B01J 27/10

(21) Application number: **86113677.8**

(22) Date of filing: **03.10.86**

(54) Carbonylation of allylic ethers to esters.

(30) Priority: **03.10.85 US 783587**
**03.10.85 US 783884**

(43) Date of publication of application:
**08.04.87 Bulletin 87/15**

(45) Publication of the grant of the patent:
**06.03.91 Bulletin 91/10**

(84) Designated Contracting States:
**BE DE FR GB IT NL**

(56) References cited:
**EP-A- 0 085 204**

**JOURNAL OF THE AMERICAN CHEMICAL SO-
CIETY, vol. 86, no. 20, 20th October 1964,
pages 4350-4353; JIRO TSUJI et al.: "Organic
syntheses by means of noble metal com-
pounds. VIII. catalytic carbonylation of allylic
compounds with palladium chloride"**

**CHEMICAL ABSTRACTS, vol. 74, no. 1, 4th
January 1971, page 269, abstract no. 12628b,
Columbus, Ohio, US; & JP-A-7028970**

(73) Proprietor: **QUANTUM CHEMICAL CORPORA-
TION (a Virginia corp.)**
**99 Park Avenue**
**New York, NY 10016(US)**

(72) Inventor: **Hanes, Ronnie M.**
**5817 Mt. Vernon Drive**
**Milford Ohio(US)**
Inventor: **Kwiatek, Jack**
**3135 North Farmcrest Drive**
**Cincinnati Ohio(US)**
Inventor: **Brima, Thomas S.**
**4320 Erie Avenue, No. 27**
**Cincinnati Ohio(US)**

(74) Representative: **Patentanwälte Grünecker,
Kinkeldey, Stockmair & Partner
Maximilianstrasse 58
W-8000 München 22(DE)**

**Description**

The present invention relates to a method for the production of esters by the reaction of allylic ethers with carbon monoxide in the presence of a metal compound as a catalyst.

Palladium chloride has been employed as a catalyst for the carbonylation of alkoxyoctadienes to alkyl nonadienoate esters; however, only low yields (less than 10%) of the ester were produced. Compounds of the palladium-type metals (i.e. the Group VIII noble metals) therefore did not appear to be good candidates for catalyzing reactions of this type even though some catalytic activity was noted with palladium chloride. The production of esters in this type of reaction on an industrial scale requires higher yields than the 10% initially observed.

US-A-3,626,005, discloses a process for the preparation of unsaturated acyl halides by carbonylating vinylic halides in the presence of a Group VIII noble metal catalyst such as palladium metal, the catalyst composition optionally containing metals such as gold, silver and copper. Additionally,

US-A-2, 876,254 also discloses a process for the preparation of esters from olefins, carbon monoxide and alcohols in the presence of a catalyst system comprising a Group VIII noble metal such as palladium and an alcohol-soluble salt of tin or germanium.

Various other U.S. patents similarly teach the production of esters such as US-A-4,172,087 in which a process for the preparation of unsaturated aliphatic esters from aliphatic dienes such as butadiene is disclosed by reacting such unsaturated components with carbon monoxide and an alcohol in the presence of a palladium catalyst and an amine base. Group VIII metal catalysts are also disclosed for the preparation of esters in a similar manner by US-A-3,161,672, US-A-3,427,344, US-A-3,652,255, US-A-3,530, 168 and US-A-3,367,961.

J.A.C.S. 86, pp. 4350-4353 (1964) discloses the carbonylation of allyl ethyl ether in ethanol as a solvent to ethyl 2-butenoate in the presence of palladium chloride as a catalyst, whereas XXIII International Conference on Coordination Chemistry, July 29 to August 3, 1984, Univ. of Colorado (Abstract of Poster Presentation TH pp. 51-56) describes the effects of solvents, catalyst promoters and inhibitors on the palladium catalyst dicarbonylation of l,4-difunctionalized-2-butenes.

None of the above references address the problems of overcoming the low yields and catalyst decomposition obtained when employing a palladium halide catalyst for the production of esters by the reaction of allylic ethers with carbon monoxide.

Additionally, in the manufacture of these esters using expensive Group VIII noble metal catalyst, it is necessary that the catalyst be recycled if it is to be employed on an industrial scale.

The ester product of the reaction may be separated from the reaction mixture by means of distillation, or vacuum distillation; however, both distillation processes require energy input which could also add to the cost of the process. Although solvent extraction processes are known in the art these known methods are not totally satisfactory for the separation of the ester from the catalyst either because of the cost of the solvents or the fact that some of the palladium catalyst is carried over into the ester that the catalyst is to be separated from.

It is the object of the present invention to provide a method for the production of esters in high yields from allylic ethers with carbon monoxide.

Said object is achieved by a method for the production of esters comprising reacting an allylic ether in with carbon monoxide in the presence of 0.005 to 1.0 mole %, based on said allylic ether, of a catalyst comprising a halide of a Group VIII noble metal, said catalyst further containing a halide compound selected from the group of hydrogen halides, carbonyl halides, acyl halides, Group IB metal halides and mixtures thereof in 5 to 50 molar excess based on said Group VIII noble metal to prevent said halide of Group VIII noble metal from being converted into said Group VIII metal during said reaction, at a temperature of from 50 to 200°C and a pressure of from 6895 to 34474 kPa to obtain an ester.

In a preferred embodiment of the invention, the reaction is conducted by dissolving the catalyst in a solvent comprising a quaternary ammonium salt or a phosphonium compound or a mixture of both that is liquid at the reaction temperature and the ester produced is separated from the catalyst by solvent extraction with a non-polar organic solvent.

In the production of esters by the reaction of an allylic ether with carbon monoxide in the presence of a Group VIII noble metal catalyst (i.e. ruthenium, rhodium, palladium, osmium, iridium and platinum and mixtures thereof) such as a palladium chloride catalyst it was observed that although the palladium chloride did in fact catalyze the reaction to the ester, the yields of the ester obtained were less than 10%. In trying to determine the cause for these low yields which are unacceptable for industrial scale reactions it was noted that during the course of the reaction the palladium chloride catalyst was unstable in that the catalyst was being converted into palladium metal. Although the prior art indicates that palladium metal would

2

EP 0 217 407 B1

catalyze the reaction of carbon monoxide with an allylic ether actual experience established that the formation of palladium metal during the carbonylation reaction detracted from the activity of the catalyst.

Means here sought to prevent the catalyst from being converted into the metal during the course of the reaction whereupon it was discovered that by employing a Group IB metal catalyst such as copper chloride (cupric or cuprous chloride) the instability of the Group VIII noble metal halides was overcome. Accordingly, the reaction is conducted in the presence of the Group VIII noble metal halide catalyst as described herein along with, for example, a Group IB metal halide which is present in an amount sufficient to prevent the catalyst from being converted into a Group VIII metal during the reaction.

The allylic ethers that may be reacted according to the method of the present invention comprise any acyclic or cyclic allylic ether having up to 20 carbon atoms and especially those having from 4 to 20 carbon atoms. In addition, the aforesaid ethers may contain up to 4 olefinically unsaturated positions and especially up to 2 olefinically unsaturated positions along an acyclic hydrocarbon chain. The esters produced have up to 21 carbon atoms and especially from 5 to 21 carbon atoms and similarly may be acyclic or may comprise an acyclic ester group attached to a cyclic group such as a cyclic hydrocarbon and have up to 4 olefinically unsaturated positions along the cyclic hydrocarbon chain and especially up to 2 of such olefinically unsaturated positions. The acyclic group of the ether or ester may be straight chain or branched chain and has from 1 to 6 and especially from 1 to 4 carbon atoms.

Examples of various allylic ethers that may be reacted according to the method of the present invention comprise:

```
8-methoxy-1,6-octadiene
methyl allyl ether
methyl-2-butenyl ether
3-methoxy-1-phenylpropene
4-methoxy-1-phenyl-2-butene
methyl 4-methoxycrotonate
1-methoxy-2-penten-4-one
ethyl allyl ether
isopropyl allyl ether
8-isopropoxy-1,6-octadiene
1-ethoxy-2-hexene
3-ethoxy-1-phenylpropene
1-methoxy-2-hexene
1,4-dimethoxy-2-butene
1-isopropoxy-2-pentene
8-phenoxy-1,6-octadiene
phenyl allyl ether
benzyl allyl ether
1-phenoxy-2-butene
1-phenoxy-2-hexene
1-phenoxy-2-penten-4-one
1-phenoxy-2-pentene
benzyl-2-butenyl ether
```

The catalyst employed for the carbonylation reaction of the present invention comprises the Group VIII noble metal halides, i.e., the halides of ruthenium, rhodium, palladium, osmium, iridium, platinum and

3

mixtures thereof, ruthenium, rhodium, palladium and platinum being preferred and palladium being especially preferred. The halides are selected from chloride, bromide and iodide and mixtures thereof, the chloride being preferred.

The halide compounds that are employed in an amount sufficient to prevent the catalyst from being converted into a Group VIII metal during the reaction comprise, for example, hydrogen chloride, hydrogen bromide, hydrogen iodide, phosgene, acyl chlorides, acyl bromides, acyl iodides, copper halides, silver halides gold halides and mixtures thereof.

In the preferred embodiment wherein acyl halides are used they include from 1 to 8 and especially 1 to 5 carbon atoms and 1 or 2 halogen atoms. Illustrative of the acyl halides used are acetyl chloride; acetyl bromide; acetyl iodide; proponyl chloride; butyryl chloride and malonyl chloride. A lower alkanol (i.e. one having from 1 to 6 carbon atoms) preferably is used with the carbonyl halide or acyl halide, most preferably with phosgene. In the preferred embodiment wherein a Group IB metal halide is utilized, the copper and silver halides are preferred with copper being particularly preferred. Gold halide is not preferred because of its cost and the difficulty in converting it into a halide such as by reacting gold metal with aqua regia. The less costly copper metal is preferred and may be used either as the cuprous halide or the cupric halide.

The catalyst is employed in the carbonylation reaction in an amount anywhere from 0.005 mole percent to 1.0 mole percent and especially from 0.05 mole percent to 0.2 mole percent based on the allylic ether employed in the reaction, this amount comprising a catalytically effective amount.

The amount of the halide compound employed in the reaction may be anywhere from 5 to 50 molar excess and especially from 10 to 30 molar excess based on the Group VIII metal, this amount comprising an amount sufficient to prevent said catalyst from being converted into said Group VIII metal during the carbonylation reaction.

The carbonylation reaction may be conducted at temperatures from 50°C to 200°C and at pressures from 6895 to 34474 kPa (1,000 psig to 5,000 psig).

In another aspect of the invention, it has been discovered that the esters produced according to the invention may be separated from the catalyst by solvent extraction when the carbonylation reaction is conducted in the presence of a solvent comprising quaternary ammonium or phosphonium compounds and mixtures thereof such as the quaternary ammonium salts and more particularly, the quaternary ammonium halides. Those quaternary ammonium salts that are liquid at the temperature of the carbonylation reaction are employed in this respect and generally comprise the tetra-alkyl ammonium halides especially the chlorides. Tetrabutyl ammonium chloride is especially suitable in this regard although other quaternary ammonium salts may be employed and include:

Tetrabutylammonium bromide

Tetradecylammonium bromide

Tetradodecylammonium bromide

Benzyldimethyl(tetradecyl)ammonium chloride

Benzyl(hexadecyl)dimethylammonium chloride

Benzyldimethyl(dodecyl)ammonium bromide

Benzyldimethyl(dodecyl)ammonium chloride

Tetrahexylammonium chloride

Benzyltributylammonium chloride

Tetradecylammonium chloride

Phosphonium compounds that may be employed

comprise:

Tetrapentylphosphonium chloride

Tetrahexylphosphonium chloride

Tetrabutylphosphonium chloride

Tetrabutylphosphonium bromide

Tetrabutylphosphonium iodide

Tetrahexylphosphonium bromide

Benzyltributylphosphonium chloride

When the carbonylation reaction is conducted in the presence of a quaternary ammonium salt or phosphonium compound, the ester obtained may be separated from the reaction mixture by means of solvent extraction whereby the ester is dissolved in a non-polar organic solvent such as a petroleum ether or the acyclic hydrocarbons and especially the aliphatic hydrocarbons having from 4 to 10 carbon atoms and especially those having from 5 to 8 carbon atoms. Either linear or branched chain acyclic hydrocarbon compounds may be employed in this respect although the linear ones are preferred. Examples of these hydrocarbons comprise pentane, hexane, heptane, octane and nonane.

Other solvents that may be used comprise 2-methyl-pentane; 2-methylhexane; 3-methylhexane; 2-methylpentane and 30-60 petroleum ether.

The following examples illustrate the invention:

EXAMPLE 1

To a 71 ml glass-lined Parr bomb was added 0.0052 g PdCl$_2$, 0.0511 g CuCl$_2$ • 2H$_2$O, 5 ml toluene and 5 ml 8-methoxy-1,6-octadiene. The bomb was purged 4 times, filled to 13790 k Pa (2,000 psig) with CO and placed in a 100° C shaker oven for 6 h. No catalyst decomposition or polymer formation was observed.

GLC analysis of the reaction product gas conducted on a Silar column and indicated 97% selectivity to methyl nonadienoate at about 48% conversion.

Thus, it can be seen that the catalyst of the present invention when employed in the carbonylation of 8-methoxy-1,6-octadiene provided excellent selectivities and yields of methyl-3,8-nonadienoates.

Comparative Example 1.

To a 71 ml glass-lined Parr bomb was added 0.0055 g PdCl$_2$, 0.0118 a CuCl$_2$ • 2H$_2$O and 5 ml 8-methoxy-1, 6-octadiene. The bomb was purged 4 times, filled to 13790 k Pa (2,000 psig) with CO and placed in a 100° C shaker oven for 6 h.A trace of a black precipitate was observed as a decomposition product. The product was analyzed as in Example 1 and 93% selectivity to methyl-3,8-nonadienoate at 8% conversion was obtained.

Comparative Example 2.

To a 71 ml glass-lined Parr bomb was added 0.0058 g PdCl₂, 0.0752 g CuCl₂ • 2H₂O, 5 ml 8-methoxy-1,6-octadiene and 2 g tetrabutylammonium chloride. The bomb was purged 4 times, filled to 13790 k Pa (2,000 psig) with CO and placed in a 100°C shaker oven for 6 h. No catalyst decomposition was observed. A GLC analysis was conducted on the product solution as in Example 1 and after the analysis, 5 ml 8-methoxy-1,6-octadiene was added to the product solution. The product solution with the 8-methoxy-1,6-octadiene was again added to a 71 ml glass-lined Parr bomb, the bomb purged 4 times and filled to 13790 k Pa (2,000 psig)with CO and placed in a 100°C shaker oven for 6 h. After the second reaction, a large amount of black precipitate was observed.

The GLC analysis of both the first and second runs indicate 92% selectivity to methyl-3,8-nonadienoate at conversions of 60% for the first run and 38% for the second run.

The foregoing example illustrates that when 0.033 mmoles PdCl₂ are employed in combination with 0.44 mmoles CuCl₂, catalyst decomposition is not minimized in the second run.

EXAMPLE 2

To a 71 ml glass-lined Parr bomb was added 0.0048 g PdCl₂, 0.0405 g anhydrous CuCl₂, 5 ml toluene and 5 ml 8-methoxy-1, 6-octadiene. The bomb was purged 4 times and filled to 13790 k Pa (2,000 psig) with CO and placed in 100°C shaker oven for 6 h. A small amount of dark precipitate was observed following the reaction. The reaction product again was analyzed by the same method employed in Example 1 and methyl-3,8-nonadienoate was obtained at 96% selectivity and 87% conversion of 8-methoxy-l,6-octadiene.

EXAMPLE 3

To a 71 ml glass-lined Parr bomb was added 0.0063 g PdCl₂, 0.0772 g CuCl₂ • 2H₂0, 1.97 g tetrabutylammonium chloride and 5 ml 8-methoxy-1, 6-octadiene. The bomb was purged 4 times, filled,13790 k Pa (2,000 psig) with CO and placed in a 100°C shaker oven for 6 h. A large amount of black precipitate was observed. The product solution was extracted with two 7 ml portions of petroleum ether and analyzed in the same manner as set forth in Example 1. Ninety-two percent selectivity to methyl-3,8-nonadienoate was observed at 70% conversion of 8-methoxy-1,6-octadiene. The catalyst residue was returned to the bomb with 5 ml 8-methoxy-l,6-octadiene and 0.0935 g CuCl₂ • 2H₂0 after which the bomb was purged 4 times and filled to 13790 k Pa (2,000 psig) with CO and placed in a 100°C shaker oven for 6 h.

The product was analyzed in the same manner as set forth in Example 1 and 89% selectivity to methyl-3,8-nonadienoate was observed at 80% conversion 8-methoxy-1,6-octadiene.

A trace of black precipitate was observed in the bottom of the bomb along with a residue. The residue was extracted three times with 10 ml petroleum ether and to the residue, extracted with the petroleum ether, was added 5 ml 8-methoxy-1,6-octadiene which again was charged to a 71 ml glass-lined Parr bomb. The bomb was purged 4 times, filled to 13790 k Pa (2,000 psig) with CO and placed in a 100°C shaker oven for 6 h. A large amount of black precipitate was observed.

The product was analyzed in the same manner as set forth in Example 1 and 86% selectivity to methyl-3,8-nonadienoate was observed at 72% conversion of 8-methoxy-1,6-octadiene.

The data of Examples 1-3 illustrate the carbonylation of 8-methoxy-1,6-octadiene to methyl-3,8-nonadienoate with palladium chloride as a catalyst. The palladium chloride is reduced during the reaction and falls out of solution; however, by the addition of cupric chloride to the reaction solution in molar excess over the palladium, the catalyst was stabilized so that high 8-methoxy-1,6-octadiene conversions may be realized.

EXAMPLE 4

Several additional reactions were conducted in 71 ml glass-lined Parr shaker bombs at 100°C, for 6 h. In each of the four reactions, 5 ml (28.6 mmoles) of 8-methoxy-l,6-octadiene was charged to the Parr bomb followed by purging four times with CO after which the bomb was pressured to 13790 k Pa (2,000 psig) with CO. The catalysts used in each instance comprise 0.005 g PdCl₂.

In the first bomb, 0.477 g CuCl₂ • 2H₂O was charged; in the second bomb, 0.477 g CuCl₂ • 2H₂O, 0.25 ml 12.5% phosgene solution in toluene was charged; in the third bomb, 0.477 g CuCl₂ • 2H₂O along with 17.9 ml acetyl chloride (0.5 mmole) was charged; and in the fourth bomb, 0.25 ml, 12.5% phosgene solution in toluene was also charged.

All reaction mixtures had small amounts of precipitate. The reaction products from each of the bombs was analyzed by GLC means employing a Silar 10 CP column and the conversion of 8-methoxy-l,6-octadiene and selectivity to methyl-3,8-nonadienoate was measured. The results obtained are listed below.

| | 8-Methoxy-1,6-octadiene | | Methyl-3,8-nonadienoate | |
|---|---|---|---|---|
| Bomb No. | Mmoles | % Conversion | Mmole | % Selectivity |
| 1 | 1.3 | 95.9 | 22.6 | 82.8 |
| 2 | 0.6 | 97.9 | 25.8 | 92.1 |
| 3 | 0.8 | 97.2 | 24.4 | 88.1 |
| 4 | 18.8 | 34.2 | 9.0 | 91.8 |

EXAMPLE 5

Four reactions were carried out in 71 ml glass-lined Parr bombs. Each bomb was charged with 2.0 g tetrabutylammonium chloride solution in water (23% water) and 0.005 g PdCl₂. The bombs were dried at 90°C in a vacuum oven (0.01 mm Hg 0,013 m bar) for 4 h. and let cool under vacuum overnight.

Each of the bombs was charged with 1 ml p-xylene (internal standard) 0.5 ml phosgene solution (12.5% in toluene) 5 ml 8-methoxy-1,6-octadiene (28.6 mmoles). Each of the bombs was charged with the following amounts of methanol: bomb 1;0.030 ml methanol (0.74 mmoles); bomb 2;0.060 ml methanol (1.5 mmoles); bomb 3;0.119 ml methanol (3.0 mmoles); bomb 4; 0.239 ml methanol (5.9 mmoles). The bombs were then purged four times with CO and pressured to 13790 k Pa (2,000 psig) with CO and placed in a shaker oven at 100°C for 3 h.

When the reaction was complete, GLC analyses of the contents of each of the bombs was conducted to determine the conversion of the 8-methoxy-1,6-octadiene and the selectivity to methyl-3,8-nonadienoate. The results obtained are tabulated below.

| | 8-Methoxy-1,6-octadiene | | Methyl-3,8-nonadienoate | |
|---|---|---|---|---|
| Bomb No. | Mmoles | % Conversion | Mmole | % Selectivity |
| 1 | 14.7 | 48.6 | 12.9 | 92.8 |
| 2 | 6.9 | 75.9 | 19.1 | 88.0 |
| 3 | 3.8 | 86.7 | 20.8 | 83.4 |
| 4 | 0.2 | 99.3 | 25.3 | 89 |

Examples 4 and 5 show that phosgene and a protic solvent such as a lower alkanol having 1 to 6 carbon atoms, e.g. methanol can be used to generate hydrogen chloride in bench-scale reactions. Conversion is dependent on the alkanol (e.g. methanol) charge even above the stoichiometric amount required to react with phosgene; selectivity is not significantly affected. Octadienyl chloride is formed in this reaction, its concentration increasing slowly to reach a constant value.

Selectivities in Examples 4 and 5 were not as high as expected in some runs. Therefore, the dependence of selectivity on ether conversion was determined. Selectivity increased with conversion to a maximum of 99% at 60% conversion, and decreased at higher conversions. This decrease resulted from further reaction of methyl nonadienoate to form ten-carbon dibasic esters.

The effect of phosgene concentration (in the range 0.02-0.08M) on reaction rate and ether selectivity was also explored. Rate, though not very sensitive to phosgene concentration in this range, was highest at about 0.05M phosgene. Maximum selectivities are also observed at this level (95% selectivity at 79% conversion; 99% selectivity at 44% conversion).

The effect of pressure in the range 3447 to 24129 k Pa (500-3500 psig) was also investigated. Maximum rate was observed at 17235 k Pa (2500 psig). Selectivity decreased from 97% to less than 90% above 20682 k Pa (3000 psig).

The data of Examples 4 and 5 also indicate that in the carbonylation of 8-methoxy-1,6-octadiene to methyl-3,8-nonadienoate with palladium chloride as a catalyst the addition of a halide compound to the reaction solution stabilizes the palladium catalyst so that palladium does not precipitate out as a metal and high 8-methoxy-1,6-octadiene conversions may be realized.

All the examples establish that the use of a quaternary ammonium salt as a solvent with the catalyst system allows removal of product ester (methyl-3,8-nonadienoate) by extraction with a non-polar organic solvent such as petroleum ether, pentane or hexane. The soluble palladium catalyst, as illustrated in the examples may then be recycled without being exposed to a distillation step.

The esters obtained according to the method of the invention may be hydrogenated and used as lubricants, plasticizers or functional fluids or may be hydrolyzed to form an acid having unsaturated groups. The acid obtained may be incorporated into polyesters manufactured from phthalic anhydride, glycols, and maleic anhydride and which are subsequently cross-linked with styrene all of which is known in the art. The unsaturated acid obtained provides a site along the polyester chain for cross-linking with styrene or equivalent monomers.

## Claims

1. A method for the production of esters comprising reacting an allylic ether with carbon monoxide in the presence of 0.005 to 1.0 mole %, based on said allylic ether, of a catalyst comprising a hal ide of a Group VIII noble metal, said catalyst further containing a hal ide compound selected from the group of hydrogen halides, carbonyl halides, acyl halides, Group IB metal hal ides and mixtures thereof in 5 to 50 molar excess based on said Group VIII noble metal to prevent said halide of Group VIII noble metal from being converted into said Group VIII metal during said reaction, at a temperature of from 50 to 200°C and a pressure of from 6895 to 34474 kPa to obtain an ester.

2. The method of claim 1 where said allylic ether has from 4 to 20 carbon atoms and said ester has from 5 to 21 carbon atoms.

3. The method of claim 1 or 2 wherein said allylic ether comprises an alkoxyalkadiene and said ester comprises an alkyl alkadienoate.

4. The method of any of claims 1 to 3 where said Group VIII metal halide and said halide compound are selected from chloride, bromide or iodide and mixtures thereof.

5. The method of claim 4 wherein said Group VIII metal comprises a member selected from ruthenium, rhodium, palladium and platinum and mixtures thereof.

6. The method of claim 4 where said Group VIII metal comprises palladium and said halide compound comprises phosgene or hydrogen chloride.

7. The method of any of claims 1 to 5 where said allylic ether comprises an alkoxyalkadiene having from 4 to 20 carbon atoms and from 1 to 6 carbon atoms in the alkoxy group and said ester comprises an alkyl alkadienoate having from 1 to 6 carbon atoms in the alkyl group.

8. The method of any of claims 1 to 5 where said allylic ether comprises 8-methoxy-1 ,6-octadiene and said ester comprises methyl-3,8-nonadienoate.

9. The method of any of claims 1 to 8 where said catalyst is dissolved in a reaction solvent selected from a quaternary ammonium salt , phosphonium compound or mixtures thereof, that is liquid at the temperature of said reaction and said ester is separated from said catalyst by solvent extraction of said ester with a non-polar organic solvent.

10. The method of any of claims 1 to 5 wherein said halide compound is selected from the group consisting of phosgene in combination with an alkanol having from 1 to 6 carbon atoms and a copper halide.

## Revendications

1. Procédé de fabrication d'esters comprenant la réaction d'un éther allylique avec du monoxyde de carbone, en présence de 0,005 a 1,0% en moles, sur la base dudit éther allylique, d'un catalyseur comprenant un halogénure d'un métal noble du Groupe VIII, ledit catalyseur contenant en outre un composé de type halogénure choisi dans le groupe des halogénures d'hydrogène, des halogénures de carbonyle, des halogénures d'acyle, des halogénures de métaux du Groupe IB, et de leurs mélanges, dans un excès de 5 à 50 fois molaire sur la base dudit métal noble du Groupe VIII, pour empêcher ledit halogénure de métal noble du Groupe VIII d'être converti en ledit métal du Groupe VIII pendant ladite réaction, à une température allant de 50 a 200° C et sous une pression allant de 6895 à 34 474 kPa, afin d'obtenir un ester.

2. Procédé selon la revendication 1, dans lequel ledit éther allylique a de 4 à 20 atomes de carbone et ledit ester a de 5 a 21 atomes de carbone.

3. Procédé selon la revendication 1 ou 2, dans lequel ledit éther allylique comprend un alcoxyalcadiène, et ledit ester comprend un alcadiénoate d'alkyle.

4. Procédé selon l'une des revendications 1 à 3, dans lequel ledix halogénure du métal du Groupe VIII et ledit composé de type halogénure sont choisis parmi le chlorure, le bromure ou l'iodure, et leurs mélanges.

5. Procédé selon la revendication 4, dans lequel ledit métal du Groupe VIII comprend un élément choisi parmi le ruthénium, le rhodium, le palladium et le platine, et leurs mélanges.

6. Procédé selon la revendication 4, dans lequel ledit métal du Groupe VIII comprend le palladium, et ledit composé de type halogénure comprend le phosgène ou le chlorure d'hydrogène.

7. Procédé selon l'une des revendications 1 à 5, dans lequel ledit éther allylique comprend un alcoxyalcadiène ayant de 4 à 20 atomes de carbone et de 1 à 6 atomes de carbone dans le groupe alcoxy, et ledit ester comprend un alcadiénoate d'alkyle ayant de 1 à 6 atomes de carbone dans le groupe alkyle.

8. Procédé selon l'une des revendications 1 à 5, dans lequel ledit éther allylique comprend le méthoxy-8 octadiène-1,6, et ledit ester comprend le nonadién-3,8 oate de méthyle.

9. Procédé selon l'une des revendications 1 à 8, dans lequel ledit catalyseur est dissous dans un solvant de réaction, choisi parmi un sel d'ammonium quaternaire, un composé de phosphonium, ou leurs mélanges, qui est liquide à la température de ladite réaction, et ledit ester est séparé dudit catalyseur par extraction par solvant dudit ester par un solvant organique non-polaire.

10. Procédé selon l'une des revendications 1 à 5, dans lequel ledit composé de type halogénure est choisi dans le groupe constitué par le phosgène en combinaison avec un alcanol ayant de 1 à 6 atomes de carbone et un halogénure de cuivre.

**Ansprüche**

1. Verfahren zur Herstellung von Estern, umfassend Umsetzen eines Allylethers mit Kohlenmonoxyd in der Gegenwart von 0,005 - 1,0 Mol%, basierend auf dem Allylether, eines Katalysators, umfassend ein Halogenid eines Gruppe VIII Edelmetalls, wobei der Katalysator weiterhin enthält eine Halogenidverbindung, ausgewählt aus der Gruppe der Halogenwasserstoffe, Carbonylhalogenide, Acylhalogenide, Gruppe IB Metallhalogenide und Mischungen davon in 5 - 50 molarem Überschuß, basierend auf der Gruppe VIII Edelmetall, um das Halogenid des Gruppe VIII Edelmetalls davor zu schützen, während der Reaktion in das Gruppe VIII Metall umgewandelt zu werden, bei einer Temperatur von 50 bis 200° C und einem Druck von 6895 bis 34474 kPa, um einen Ester zu erhalten.

2. Verfahren nach Anspruch 1,
**dadurch gekennzeichnet,**
daß der Allyläther 4 bis 20 Kohlenstoffatome besitzt, und der Ester 5 bis 21 Kohlenstoffatome besitzt.

3. Verfahren nach einem der Ansprüche 1 oder 2,
**dadurch gekennzeichnet,**
daß der Allylether ein Alkoxyalkadien enthält, und der Ester ein Alkylalkadienoat enthält.

4. Verfahren nach einem der spruche 1 bis 3,
**dadurch gekennzeichnet,**
daß das Gruppe VIII Metallhalogenid und die Halogenidverbindung ausgewählt werden aus Chlorid, Bromid oder Iodid und Mischungen davon.

5. Verfahren nach Anspruch 4,
**dadurch gekennzeichnet,**
daß das Gruppe VIII Metall umfaßt ein Mitglied ausgewählt aus Ruthenium, Rhodium, Palladium und Platin und Mischungen davon.

6. Verfahren nach Anspruch 4,
**dadurch gekennzeichnet,**
daß das Gruppe VIII Metall umfaßt Palladium, und die Halogenidverbindung umfaßt Phosgen oder Chlorwasserstoff.

7. Verfahren nach einem der Ansprüche 1 bis 5,
**dadurch gekennzeichnet,**
daß der Allylether umfaßt ein Alkoxyalkadien mit 4 bis 20 Kohlenstoffatomen und mit 1 bis 6 Kohlenstoffatomen in der Alkoxygruppe, und der Ester umfaßt ein Alkylalkadienoat mit 1 bis 6 Kohlenstoffatomen in der Alkylgruppe.

8. Verfabren nach einem der Ansprüche 1 bis 5,
**dadurch gekennzeichnet,**
daß der Allylether umfaßt 8-Methoxy-1,6-oktadien,und der Ester umfaßt Methyl-3,8-nonadienoat.

9. Verfahren nach einem der Ansprüche 1 bis 8,
**dadurch gekennzeichnet,**
daß der Katalysator in einem Reaktionslösungsmittel gelöst ist, ausgewählt aus einem quartären Ammoniumsalz, einer Phosphonium-Verbindung oder Mischungen davon, welches bei der Reaktionstemperatur flüssig ist, und der Ester von dem Katalysator getrennt wird durch Lösungsmittelextraktion des Esters mit einem nichtpolaren organischen Lösungsmittel.

10. Verfahren nach einem der Ansprüche 1 bis 5,
**dadurch gekennzeichnet,**
daß die Halogenidverbindung ausgewählt wird aus der Gruppe bestehend aus Phosgen in Verbindung mit einem Alkanol mit 1 bis 6 Kohlenstoffatomen und einem Kupferhalogenid.